(19) Europäisches Patentamt
European Patent Office
Office européen des brevets

(11) **EP 1 054 897 B1**

(12) **FASCICULE DE BREVET EUROPEEN**

(45) Date de publication et mention
de la délivrance du brevet:
**02.08.2006 Bulletin 2006/31**

(51) Int Cl.:
*C07K 14/47* *(2006.01)*    *G01N 33/68* *(2006.01)*

(21) Numéro de dépôt: **99903764.1**

(22) Date de dépôt: **16.02.1999**

(86) Numéro de dépôt international:
**PCT/FR1999/000338**

(87) Numéro de publication internationale:
**WO 1999/041280 (19.08.1999 Gazette 1999/33)**

(54) **PROCEDE DE PURIFICATION DE LA PrPres A PARTIR D'UN ECHANTILLON BIOLOGIQUE ET SES APPLICATIONS**

VERFAHREN ZUR REINIGUNG VON PRPRES AUS EINEM BIOLOGISCHEN MUSTER UND DESSEN VERWENDUNG

METHOD FOR PURIFYING PrPres FROM A BIOLOGICAL SAMPLE AND APPLICATIONS

(84) Etats contractants désignés:
**AT BE CH DE DK ES FI FR GB GR IE IT LI LU NL PT SE**

(30) Priorité: **16.02.1998 FR 9801823**

(43) Date de publication de la demande:
**29.11.2000 Bulletin 2000/48**

(73) Titulaire: **COMMISSARIAT A L'ENERGIE ATOMIQUE**
**75015 Paris (FR)**

(72) Inventeur: **DESLYS, Jean-Philippe**
**F-78150 Le Chesnay (FR)**

(74) Mandataire: **Vialle-Presles, Marie José et al**
**Cabinet ORES,**
**36,rue de St Pétersbourg**
**75008 Paris (FR)**

(56) Documents cités:
**WO-A-98/30909**

• **S LEHMANN & D A HARRIS: "Two mutant prions expressed in cultured cellsacquire biochemical properties reminiscentof the scrapie isoform." PROCEEDINGS OF THE NATIONAL ACADEMY OF SCIENCES OF USA., vol. 93, mai 1996, pages 5610-5614, XP002084017 WASHINGTON US**
• **S G CHEN ET AL.: "Allelic origin of the abnormal prion protein isoform in familial prion disease" NATURE MEDICINE., vol. 3, no. 9, septembre 1996, pages 1009-1015, XP002084018 CO US**
• **File Medline, abstract no. 92046319, 1992 XP002084019 & B CAUGHEY ET AL.: "N-terminal truncation of the scrapie-associated form of PrP by lysosomal protease (s): implications regarding the conversion of PrP to the protease-resistant state" JOURNAL OF VIROLOGY., vol. 65, no. 12, décembre 1991, pages 6597-6603,**
• **File Medline, abstract 91140725, 1991 XP002084020 & M P MC KINLEY ET AL.: "Scrapie prion rod formation in vitro requires both detergent extraction and limited proteolysis" JOURNAL OF VIROLOGY, vol. 65, no. 3, mars 1991, pages 1340-1351,**

## Description

La présente invention est relative à un nouveau procédé de purification de la PrPres à partir d'un échantillon biologique en vue de son utilisation pour la détection qualitative et/ou quantitative de la PrPres dans ledit échantillon.

Les encéphalopathies subaiguës spongiformes transmissibles sont provoquées par des agents transmissibles non conventionnels (ANTC), encore appelés prions, dont la nature précise demeure inconnue à ce jour. Les ESST comprennent essentiellement la maladie de Creutzfeldt-Jakob, chez l'homme (MCJ ou CJD pour Creutzfeldt-Jakob *disease),* la tremblante, chez le mouton et la chèvre et l'encéphalopathie spongiforme bovine (ESB ou BSE pour *bovine spongiform encephalopathy*), chez les bovins ; d'autres encéphalopathies ont été mises en évidence chez le vison ou certains animaux sauvages, tels que le cerf et l'élan.

Ces maladies sont d'évolution constamment fatale et il n'existe, à l'heure actuelle, aucun traitement efficace.

Dans les encéphalopathies subaiguës spongiformes transmissibles, il existe une accumulation d'une protéine de l'hôte, la PrP (ou protéine du prion), sous une forme anormale (PrPres), principalement dans le système nerveux central ; la PrPres copurifie avec l'infectiosité et son accumulation précède l'apparition des lésions histologiques. *In vitro,* elle est toxique pour des cultures de neurones.

Deux propriétés biochimiques permettent habituellement de distinguer la PrPres de la PrP normale : la PrPres est partiellement résistante aux protéases et est insoluble dans les agents tensioactifs anioniques.

Pour pouvoir détecter la PrPres présente dans un échantillon, il est nécessaire de soumettre ce dernier à différentes opérations, pour enrichir ledit échantillon en PrPres. tout en éliminant la PrP normale, de manière à ce que la PrPres puisse ensuite être détectée par toute méthode spécifique appropriée, sans entrainer :

.  de faux-positifs, dus à une présence de PrP normale ou d'autres contaminants, ou
.  de faux-nésatifs, dus à une concentration insuffisante de PrPres dans l'échantillon biologique final.

Dans ce but un certain nombre de procédés d'isolement et/ou de purification de la PrPres ont été proposés. Ils sont essentiellement basés sur la méthode mise au point par Hilmert et Diringer (Nature, 1983, 306, 476-478) et comprennent, de manière générale, une extraction par un détergent, des ultracentrifugations différentielles et un traitement par des enzymes protéolytiques (Multhaup G. et al., EMBO J., 1985, 4, 6, 1495-1501 ; Takahashi K. et al., Microbiol. Imunol., 1986, 30, 2, 123-131 ; Hope J. et al., EMBO J., 1986, 5. 10, 2591-2597 . Grathwohl KUD et al., Arch. Virol., 1996, 141, 1863-1874 ; Kascsak RJ et al., Immunol. Investig.,

1997, 26, 259-268 ; R.E. Race et al., J. Gen. Virol., 1992, 73, 3319-3323 ; Doi et al., J. Gen. Virol., 1988, 69, 955-960 ; T. Muramoto et al., Am. J. Pathol. 1993, 143, 5, 1470-1479 ; Farquhar C.F. et al., Gen. Virol., 1994, 75, 495-504 et J. Gen. Virol., 1996, 77, 1941-1946). Le document Nature Medecine, vol. 3, n° 9, 1996, Chen et al., p. 1009-1015 décrit une méthode de purification de la PrPres comprenant plusieurs cycles d'extraction par un détergent (sarkosyl), suivi d'une centrifugation différentielle, une ultracentrifugation, et un traitement par une enzyme protéolytique. Le document Cell, vol. 86, 1996, Thuret et al., p. 565-576 décrit une méthode d'isolement de la PrPres qui comprend une extraction par un détergent et une ultracentrifugation. Ces procédés ont l'inconvénient de comporter un nombre important d'étapes incluant plusieurs ultracentrifugations, qui sont lourdes à mettre en oeuvre et entraînent des pertes cumulatives de PrPres, qui conduisent alors à une sensibilité insuffisante pour obtenir un seuil de détection et une quantification fines de la PrPres.

Ces différentes méthodes nécessitent un appareillage de laboratoire de recherche et des temps de mise en oeuvre, qui ne sont pas compatibles avec une utilisation sur le terrain, en particulier dans les abattoirs.

Or, il existe un besoin de vérification rapide de l'absence ou de la présence d'une encéphalopathie subaiguë spongiforrne transmissible, au moment de l'abattage de l'animal.

En conséquence, l'Inventeur s'est donné pour but de pourvoir à un procédé de purification d'un échantillon biologique en vue de son utilisation pour une détection rapide et fiable de la PrPres, qui soit suffisamment simple à mettre en oeuvre pour qu'il puisse être utilisé sur le terrain, et notamment dans les abattoirs et qui réponde ainsi mieux aux besoins de la pratique que les procédés de l'Art antérieur.

En effet, le procédé selon l'invention :

-    est simple à mettre en oeuvre,
-    est fiable
-    est facile à interpréter : il augmente le seuil de sensibilité de détection de la PrPres, en éliminant les faux-positifs (PrP normale et autres contaminants)

et il élimine les faux-négatifs, car il permet d'obtenir, en valeur absolue, une quantité importante de PrPres, puisqu'il est possible de traiter des quantités importantes de matériel biologique avec un rendement de purification supérieur à 80 % ; ceci est particulièrement intéressant dans les abattoirs et fournit des échantillons dans lesquels la PrPres est facilement détectable avec des tests de diagnostic usuels.

La présente invention a pour objet un procédé de purification de la PrPres à partir d'un échantillon biologique, caractérisé en ce qu'il comprend essentiellement :

(1) l'incubation pendant 30 secondes à 2 heures, de préférence pendant 30 secondes à 10 minutes, à une température inférieure à 80°C, dudit échantillon biologique avec un tampon A comprenant au moins un agent tensioactif, en quantité comprise entre le quart et quatre fois, de préférence entre le quart et une fois et demi le poids de l'échantillon biologique et éventuellement l'incubation préalable, postérieure ou simultanée avec une protéase, pour former une suspension micellaire ou lamellaire S1 opalescente à trouble ; n'importe quel agent tensioactif ou mélange d'agents tensioactifs, dans les conditions de température et de quantités précitées ne solubilisent pas la majorité de la PrPres, qui reste en suspension, alors que la PrP normale est solubilisée, voire détruite, en cas d'adjonction de protéase ; ladite incubation est de préférence réalisée à une température inférieure à 50°C. en présence d'une quantité d'agent tensioactif comprise entre le quart et une fois et demi le poids de l'échantillon biologique ; conformément à l'invention, la protéase peut en effet être ajoutée soit avant l'adjonction de l'agent tensioactif, soit après, soit simultanément :

(2) l'addition, à ladite suspension micellaire ou lamellaire S1 obtenue en (1) d'un tampon B en quantité apte à éclaircir ladite suspension (formation d'une microémulsion ou d'une microsuspension, par exemple), lequel tampon B est constitué d'un solvant ou d'un mélange de solvants, qui ne solubilise pas la PrPres et présente une constante diélectrique comprise entre 10 et 25 : on obtient ainsi une suspension S2 limpide à l'oeil nu ;

(3) la centrifugation de la suspension S2 obtenue à l'étape (2) ; ladite centrifugation est par exemple effectuée pendant 2 à 10 minutes. à une vitesse inférieure à 20 000 g. de préférence à une vitesse comprise entre 3 500 g et 17 500 g ; la PrPres se retrouve dans le culot de centrifugation avec un rendement de purification en PrPres compris, de manière surprenante, entre 80 et 100 % ; de manière avantageuse, le temps et la vitesse de centrifugation peuvent être adaptés pour aboutir au même résultat, à savoir l'obtention d'un rendement de purification en PrPres compris entre 80 et 100 % et

(4) la solubilisation dudit culot dans un tampon C comprenant au moins un agent tensioactif, tel que défini à l'étape (1), à une concentration comprise entre 0,1 % et 5 %, de préférence comprise entre 0.25 % et 1 %, par rapport au volume de tampon C (p/v) et/ou au moins un agent chaotrope à une concentration comprise entre 0.1 M et 8 M et à une température comprise entre la température ambiante et 100°C, de préférence égale ou supérieure à 80°C ; dans de telles conditions de température, les agents tensioactifs précités, de préférence les agents tensioactifs ioniques, et/ou les agents chaotropes solubilisent la PrPres.

Lesdites étapes (1) et (2) peuvent être réalisées simultanément ou successivement ; de préférence, elles sont réalisées successivement.

Selon un mode de mise en oeuvre avantageux dudit procédé, lorsque l'échantillon biologique est un tissu ou un organe, ce dernier est, préalablement à l'étape (1), homogénéisé, par exemple par broyage mécanique, dans un tampon d'homogénéisation, constitué d'un tampon neutre, tel que l'eau ou d'un tampon isotonique, tel que le glucose à 5 %.

Selon un autre mode de mise en oeuvre avantageux dudit procédé, la température mise en oeuvre à l'étape (1) est comprise entre la température ambiante et 50°C ; elle est de préférence de 37°C.

De manière préférée, le tampon A comprend un agent tensioactif sélectionné dans le groupe constitué par :

- des tensioactifs anioniques, tels que le SDS (dodécylsulfate de sodium), le sarkosyl (lauroyl sarcosine), le cholate de sodium, le désoxycholate de sodium, le taurocholate de sodium.
- des tensioactifs zwitterioniques, tels que le SB 3-10 (décyl sulfobétaïne), le SB 3-12 (dodécyl sulfobétaïne), le SB 3-14, le SB 3-16 (hexadécyl sulfobétaïne), le CHAPS et le désoxyCHAPS.
- des tensioactifs non-ioniques, tels que le C12E8 (dodécyl octaéthylène glycol), le Triton X100. le Triton X114, le Tween 20 le Tween 80, le MEGA 9 (nonanoyl méthyle glucamine), l'octylglucoside, le LDAO (dodécyl diméthylamine oxyde) ou le NP40 ou
- des mélanges de tensioactifs tels qu'un mélange d'un agent tensioactif ionique et d'un agent tensioactif non-ionique et notamment le mélange SDS-Tween 80 ou le mélange sarkosyl-Triton X100 ou le mélange de deux agents tensioactifs ioniques, tel que le mélange SDS-désoxycholate ou d'un mélange d'un agent tensioactif ionique et d'un agent tensioactif zwitterionique.

Selon un autre mode de mise en oeuvre avantageux dudit procédé, le tampon B est de préférence sélectionné parmi les alcools en $C_3$-$C_6$ et les mélanges d'alcool dont la constante diélectrique théorique moyenne est comprise entre 10 et 25. Les alcools ou mélanges d'alcools suivants sont particulièrement préférés : butanol-1, butanol-2, méthyl-2 propanol-1, isopropanol isopropanol + pentanol, éthanol + hexanol, butanol + pentanol, etc...

On entend par constante diélectrique, au sens de la présente invention, la constante diélectrique statique ε, mesurée dans des champs statiques ou à fréquence relativement faible ; elle correspond au rapport déplacement électrique D/force du champ électrique E. lorsque un champ électrique est appliqué à la solution, à une température comprise entre 293,15 et 298,15 K.

La constante diélectrique des liquides, telle que définie ci-dessus, est plus particulièrement décrite dans CRC Handbook of Chemistry and Physics (Ed. David R.

Lide, 75ème édition. 1994, CRC Press).

Pour un mélange de solvants, on entend par constante diélectrique théorique moyenne, la moyenne des constantes diélectriques de chaque solvant, pondérée par sa proportion dans le mélange.

L'addition du tampon B à l'étape (2) permet, de manière surprenante, d'obtenir des rendements de purification supérieurs à 90 %, avec des conditions de centrifugation à faible vitesse : elle permet tout en maintenant un rendement important, de diminuer de manière significative la quantité de culot final : de manière avantageuse, la quantité de culot final est de préférence inférieure à 10 % du poids d'échantillon biologique de départ pour permettre effectivement de l'utiliser dans le cadre d'un dosage immunologique, alors que lorsqu'on ne rajoute que du tampon A. on se situe dans les conditions de l'Art antérieur, qui imposent une ultracentrifugation pour obtenir des rendements suffisant en PrPres, en vue d'une détection.

On peut noter que l'on peut également obtenir des rendements de purification supérieurs à 80 %, en faisant varier le temps et la vitesse de centrifugation : 2 à 10 minutes à une vitesse inférieure à 20 000 g ou pendant une durée diminuée proportionnellement à l'augmentation du nombre de g.

De manière préférée, le rapport rendement en PrPres dans la phase solide/quantité de culot récupéré après centrifugation de la suspension S2 est supérieur à 10, lorsque l'échantillon de départ correspond à 100 mg de cerveau.

Egalement de manière préférée le tampon C mis en oeuvre à l'étape (4) comprend un agent chaotrope, qui est notamment sélectionné dans le groupe constitué par l'urée et la guanidine ou un mélange de ceux-ci : tout autre agent chaotrope peut également être utilisé.

De préférence, l'urée est à une concentration comprise entre 0,25 et 8 M et la guanidine est à une concentration comprise entre 0,1 et 6 M.

Lorsque le tampon C est un mélange d'au moins un agent tensioactif et d'au moins un agent chaotrope, il est sélectionné, de manière préférée, dans le groupe constitué par les mélanges suivants : mélange SDS et urée, mélange sarkosyl et urée, mélange désoxycholate et urée ou mélange sarkosyl et guanidine et sarkosyl, guanidine et urée. De manière préférée, dans le mélange SDS et urée, le SDS est à 0,25-1 % et l'urée est à 0,25-6 M : dans le mélange sarkosyl et urée, le sarkosyl est à une concentration comprise entre 0.25 et 1 %, et l'urée est à une concentration comprise entre 0,25 et 8 M ; dans le mélange sarkosyl et guanidine, le sarkosyl est à une concentration comprise entre 0,25 et 1 % et la guanidine est à une concentration comprise entre 0,5 M - 3 M et dans le mélange sarkosyl, guanidine et urée, le sarkosyl est à une concentration comprise entre 0.25 et 1 %, la guanidine est à une concentration comprise entre 0,5 M - 3 M et l'urée est à une concentration comprise entre 2 et 6 M.

Le tampon de Laemmli (SDS à 4 %. Tris-HCl 0.1 M pH 8, saccharose à 5 % et β-mercaptoéthanol à 2 %) peut également être utilisé, notamment pour les *Western blot.*

La présente invention a également pour objet un procédé de détection de la PrPres dans un échantillon biologique, caractérisé en ce qu'il comprend :

- le traitement dudit échantillon tel que défini ci-dessus.
- la dilution de l'échantillon obtenu si nécessaire et
- la détection de la PrPres par toute méthode analytique appropriée, telle qu'une méthode immunologique (ELISA, *Western blot),* produisant un signal spécifique.

L'étape de dilution précitée permet de neutraliser le tampon C, en vue d'une détection de la PrPres par une méthode ELISA ; elle est par exemple réalisée avec un tampon comprenant de l'albumine, conduisant à une concentration finale en albumine comprise entre 2 et 10 % (p/v) ou avec un tampon à base de désoxycholate, à 1 %, par exemple.

Un échantillon biologique ainsi traité contient une concentration efficace en PrPres, de manière à ce que cette dernière puisse être détectée par toute méthode analytique, notamment une méthode immunologique, directement dans cet échantillon,

En variante, la présente invention a pour objet un procédé de purification de la PrPres à partir d'un échantillon biologique, caractérisé en ce qu'il comprend essentiellement :

(1) l'incubation, pendant 30 secondes à 2 heures, de préférence pendant 30 secondes à 10 minutes à une température inférieure à 80°C dudit échantillon biologique avec un tampon A comprenant au moins un agent tensioactif, en quantité comprise entre le quart et quatre fois, de préférence entre le quart et une fois et demi le poids de l'échantillon biologique et éventuellement l'incubation préalable, postérieure ou simultanée avec une protéase, pour former une suspension micellaire ou lamellaire S1 opalescente à trouble ; conformément à l'invention la protéase est en effet ajoutée soit avant l'adjonction de l'agent tensioactif soit après, soit simultanément :

(2) l'addition. à ladite suspension micellaire ou lamellaire S1 obtenue en (1) d'un tampon B en quantité apte à former une séparation de phase, lequel tampon B est constitué d'un solvant ou d'un mélange de solvants, qui ne solubilise pas la PrPres et présente une constante diélectrique comprise entre 10 et 25 :

(3) la centrifugation de la suspension obtenue à l'étape (2) : ladite centrifugation est par exemple effectuée pendant 2 à 10 minutes, à une vitesse inférieure à 20 000 g, de préférence à une vitesse comprise entre 3 500 g et 17 500 g : la PrPres se retrouve à l'interface :

(4) la récupération du film présent à l'interface ;

(5) la resolubilisation dudit film avec un tampon A sans protéase.

(6) la centrifugation de la suspension obtenue à l'étape (5), pendant 2 à 10 minutes, à une vitesse inférieure à 20 000 g, de préférence à une vitesse comprise entre 3 500 g et 17 500 g ; la PrPres se retrouve dans le culot de centrifugation, avec un rendement de purification en PrPres compris, de manière surprenante, entre 70 et 100 % ; de manière avantageuse, le temps et la vitesse de centrifugation peuvent être adaptés pour aboutir au même résultat, à savoir l'obtention d'un rendement de purification en PrPres compris entre 70 et 100 % et

(7) la solubilisation dudit culot dans un tampon C comprenant au moins un agent tensioactif, tel que défini à l'étape (1), à une concentration comprise entre 0.1 % et 5 %, de préférence comprise entre 0.25 % et 1 %, par rapport au volume de tampon C (p/v) et/ou au moins un agent chaotrope à une concentration comprise entre 0,1 M et 8 M et à une température comprise entre la température ambiante et 100°C, de préférence égale ou supérieure à 80°C: dans de telles conditions de température, les agents tensioactifs précités, de préférence les agents tensioactifs ioniques, et/ou les agents chaotropes solubilisent la PrPres.

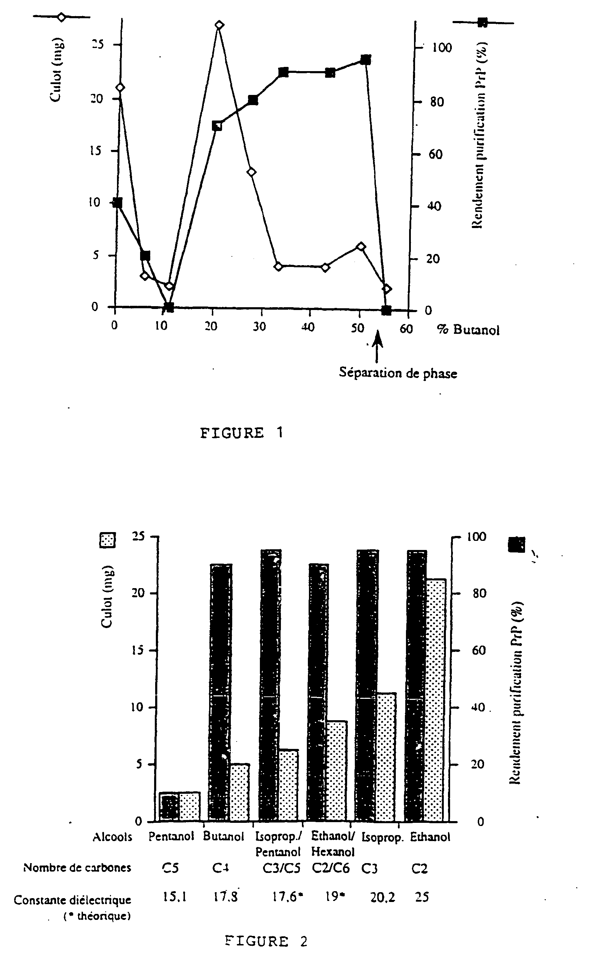

Les quantités de tampon B à ajouter pour obtenir une microémulsion, une microsuspension ou une séparation de phase sont établies à l'aide d'une gamme de tampon B. comme illustré pour le butanol-1, à la figure 1 : elles peuvent varier en fonction du tampon A et des constituants sélectionnés pour le tampon B.

En variante, l'étape de solubilisation (étape (4) du premier procédé ou étape (7) du deuxième procédé) dans ledit un tampon C comprend le chauffage à une température égale ou supérieure à 80°C. pendant à 10 minutes, suivie d'une centrifugation de préférence pendant 2 à 10 minutes, à une vitesse inférieure à 20 000 g, de préférence à une vitesse comprise entre 3 500 g et 17 500 g ; dans ce cas, la PrPres est resolubilisée et se trouve dans le surnageant : dans de telles conditions, on obtient un échantillon particulièrement bien adapté à un dosage de la PrPres par une méthode ELISA.

La présente invention a également pour objet un kit de traitement d'un échantillon biologique, caractérisé en ce qu'il comprend outre un tampon d'homogénéisation dudit échantillon biologique, des quantités convenables de tampon A, de tampon B et de tampon C, tels que définis ci-dessus.

La présente invention a également pour objet un kit de détection de la PrPres, caractérisé en ce qu'il comprend des quantités convenables de tampon d'homogénéisation de l'échantillon biologique dans lequel la PrPres doit être détectée, des quantités convenables de tampon A, de tampon B et de tampon C tels que définis ci-dessus, ainsi qu'au moins un anticorps anti-PrPres convenable.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

- la figure 1 illustre l'influence de la quantité de tampon B (butanol) sur le rendement de purification de la PrPres (en %) et la quantité de culot obtenu après centrifugation;
- la figure 2 illustre l'influence de différents tampons B sur le rendement de purification de la PrPres (en %) et la quantité de culot obtenu après centrifugation :
- la figure 3 illustre le rôle de la constante diélectrique théorique moyenne, telle que définie ci-dessus, pour comparer différents mélanges d'alcools utilisés comme tampon B ;
- la figure 4 illustre un exemple de détection de la PrPres par *Western blot :*
- la figure illustre la sensibilité et le rendement du test ELISA sur différentes dilutions d'échantillons traités, conformément à l'invention :
- la figure 6 illustre un test ELISA effectué directement sur des homogénats, et
- la figure 7 illustre une comparaison de différents tampons C.

Il doit être bien entendu, toutefois, que ces exemples sont donnés uniquement à titre d'illustration de l'objet de l'invention, dont ils ne constituent en aucune manière une limitation.

**EXEMPLE 1 : Traitement d'un échantillon de cerveau bovin pour le dosage de la PrPres sur le terrain : sélection de la quantité de tampon B convenable.**

- On prélève 500 mg de cerveau bovin : on le broie et on l'homogénéise à 25 % (p/v) dans une solution de glucose à 5 %.
  Pour réaliser l'homogénéisation, le prélèvement de cerveau (500 mg) et 1 ml de glucose sont introduits dans des tubes comprenant des billes en céramique, sous agitation. On récupère le surnageant (environ 1,5 ml) ; les billes sont rincées en suspension dans 500 μl de glucose et agitées : on récupère le surnageant obtenu qui est mélangé avec le surnageant précédent (2 ml).
- On incube 400 μl d'homogénat tel qu'obtenu ci-dessus (équivalent à 100 mg de cerveau/avec 400 μl de tampon A comprenant un mélange à part égale à 25 % (p/v) de SDS et à 25 % (v/v) de Tween 80 dans un rapport 1/1 (v/v) ainsi que de la protéinase K (0.1 mg/ml de tampon A, soit 0,4 mg/g tissu) pendant 10 minutes (2 fois 5 min) à 37°C (étape (1)). En variante, le tampon A comprend avantageusement du sarkosyl à 10 % et du Triton X100 à 10 % : ce dernier tampon A. qui ne comprend pas de SDS est plus avantageux, car il n'entraîne pas de perturbation dans la détection de la PrPres par des anticorps spécifiques, par une méthode de type ELISA.

- On ajoute 0 à 1 000 μl de butanol (tampon B) (étape (2)).

La figure 1 montre le rendement de purification en PrPres (%), quantifié en *Western blot* et la quantité de culot (en mg) en fonction de la quantité de tampon B ajoutée.

Cette figure montre qu'entre 10 et 53 %, la PrPres est maintenue en suspension et qu'entre 30 et 50 % de butanol, on obtient un rendement de purification de l'ordre de 100 % et un culot inférieur à 10 mg.

- On centrifuge à 3 800 g (4 000 RPM, centrifugeuse JOUAN) pendant 10 minutes (étape (3)) ; on élimine le surnageant et on dissout le culot obtenu qui contient la PrPres avec 80-100 μl d'un tampon C (étape (4)) comprenant :

> . soit du SDS à 0,5 % et de l'urée 0,5 M.
> . soit un tampon de Laemmli.
> . soit du sarkosyl à 0,5 % et de l'urée 6M

pendant 5 minutes à 100°C.

Ce traitement permet la dissolution de la PrPres : l'échantillon est directement prêt à être utilisé dans un dosage de type immunologique tel que ELISA ou *Western blot.*

Pour réaliser un ELISA. le culot est de préférence dissous dans un tampon C comprenant du sarkosyl (0,25-1 %) et de l'urée (0,25-8M) ou du SDS (0,25-1 %) et de l'urée (0,25-1 M) ; l'échantillon obtenu sera de préférence dilué (au ¼ ou au ½), après chauffage, avec un tampon contenant de l'albumine conduisant à une concentration finale en albumine comprise entre 2 et 10 % (p/v) ou avec un tampon contenant du désoxycholate à 1 %.

En variante, l'échantillon dilué est chauffé à 100°C pendant 5-10 minutes puis centrifugé pendant 2 à 10 minutes, à une vitesse inférieure à 20 000 g, de préférence à une vitesse comprise entre 3 500 g et 17 500 g ; le surnageant est dilué entre le ¼ et le ½ avec un tampon ELISA.

Dans le cas présent, la quantification de la PrPres est réalisée en *Western blot,* par mélange vol/vol avec du tampon de Laemmli : l'échantillon dilué est ensuite déposé sur gel pour une détection par *Wesrern blot ;* les quantités de PrPres détectées sont rapportées à une gamme linéaire de dilutions de PrPres purifiée dans les mêmes conditions que ci-dessus. à partir d'un même homogénat de cerveau de vache atteinte d'ESB. au stade terminal de la maladie (contrôle positif).

Les échantillons traités conformément au procédé selon l'invention, sont débarrassés du bruit de fond et permettent un dosage fiable, spécifique et quantitatif de la PrPres.

Le procédé selon l'invention permet d'augmenter le rendement de purification en PrPres, de manière significative :

- en effet, lorsque l'on ne rajoute que du tampon A. le rendement en PrPres n'est que de l'ordre de 40 % ; on observe donc une perte de l'ordre de 60 % de la PrPres, notamment du fait de sa répartition entre la phase solide et la phase liquide ; de plus, le culot est important. Ceci explique pourquoi dans l'Art antérieur, les protocoles décrits utilisent le sarkosyl qui entraîne l'obtention de culots plus faibles, mais qui impose des ultracentrifugations lourdes pour avoir un rendement suffisant.

- l'étape (2) permet d'augmenter le rendement en PrPres dans la phase solide : on obtient un rendement de l'ordre de 80-100 % dans la phase solide de la suspension S2, tout en diminuant la quantité de culot, comme précisé ci-dessus.

**EXEMPLE 2 ; Traitement d'un échantillon de cerveau bovin pour le dosage de la PrPres sur le terrain : variante de l'étape (1) et de l'étape (2).**

- l'étape d'homogénéisation est identique à celle décrite à l'exemple 1.
- ensuite, on incube les 2 ml d'homogénat obtenus à partir des 500 mg de cerveau bovin, avec 2 ml de tampon A, dans les mêmes conditions que celles exposées à l'exemple 1 (étape (1)).
- on ajoute 3 ml de tampon B, dans les mêmes conditions que celles exposées à l'exemple 1 (étape (2)).
- la suite du procédé est identique à celui de l'exemple 1.

**EXMPLE 3: Traitement d'un échantillon de cerveau bovin pour le dosage de la PrPres sur le terrain : variante de l'étape d'homozénéisation.**

- On prélève 250 mg de cerveau bovin : on le broie et on l'homogénéise à 25 % (p/v) dans une solution de glucose à 5 %.

Pour réaliser l'homogénéisation, le prélèvement de cerveau (250 mg) et 750 μl de glucose sont introduits dans des tubes comprenant des billes en céramique, sous agitation, pendant 40 secondes (appareil RIBOLYSER-HYBAID) : on prélève 400 μl de surnageant puis on procède comme à l'exemple 1.

**EXEMPLE 4 : Comparaison de différents tampons B sur le rapport rendement de purification en PrPres/ quantité de culot.**

L'échantillon est traité dans les mêmes conditions que celles exposées à l'exemple 1, à l'exception de la quantité de tampon B qui est de 600 μl.
La figure 2 illustre les rapports obtenus en *Western blot* avec différents tampons B : pentanol, butanol, mélange isopropanol + pentanol, mélange éthanol + hexanol, isopropanol et éthanol ; les mélanges ont été réalisés vol/vol.

**EXEMPLE 5 : Comparaison des constantes diélectriques de différents mélanges et leur influence sur le rendement de purification en PrPres et la quantité de culot.**

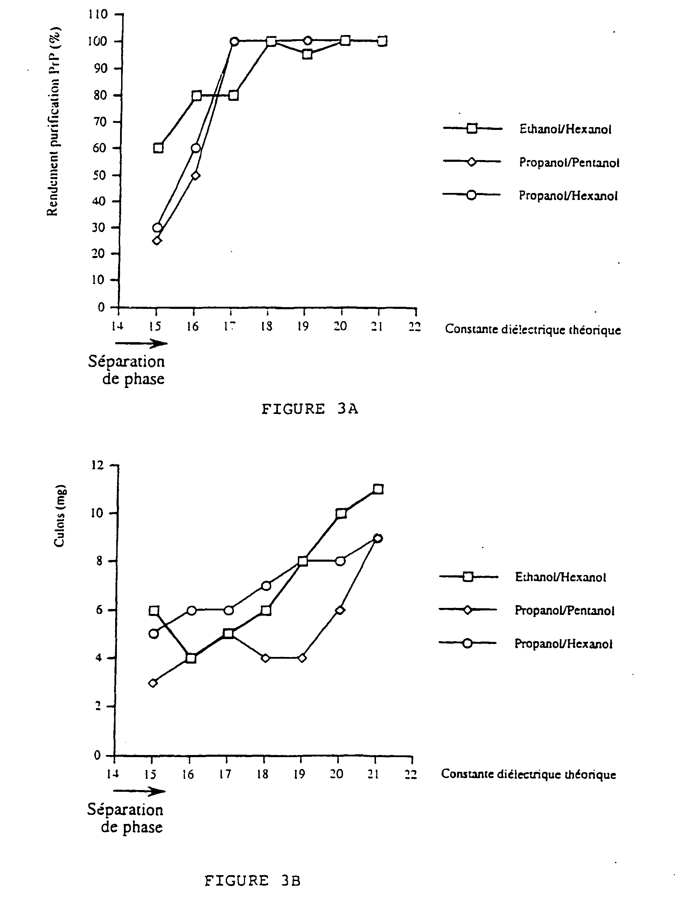

Le procédé est réalisé dans les conditions exposées à l'exemple 4. La figure 3 illustre les résultats obtenus : dans le cas où le tampon B est un mélange d'alcools, le volume de chaque alcool est calculé en fonction de sa constante diélectrique et de la constante diélectrique théorique du mélange souhaitée (par exemple 17) et rapporter à 600 μl de volume total d'alcool. Par exemple, pour le mélange hexanol/éthanol on obtient la formule suivante :

$$13.y + 25 (1-y) = 17$$

y représentant le pourcentage d'hexanol. 13 étant la constante diélectrique de l'hexanol et 25 étant la constante diélectrique de l'éthanol.
Pour une constante diélectrique théorique moyenne de 15 ou moins, on observe une séparation de phase.

**EXEMPLE 6 : Détection de la PrPres par *Western blot.***

**\* Protocole :**

1) incubation à 37°C, 2 fois 5 min de 400 mg d'homogénat de cerveau de vache à 25 % (poids/volume) et de 400 μl de tampon A comprenant 400 μl d'un mélange à part égale (v/v) de SDS à 25 % (p/v) et de Tween 80 à 25 % (v/v) (50/50) et de protéinase K (PK) à 0.1 mg/ml de tampon A.
2) on ajoute 600 μl de tampon B (sauf échantillons des pistes 7 et 8 : 1 000 μl tampon B), constitué de butanol-1.
3) on centrifuge à 15000 RPM pendant 5 min (environ 17 000 g).
4) le culot de centrifugation est repris dans 100 μl

de tampon de Laemmli contenant 4 % de SDS et chauffé à 100°C pendant 5 min.

*\*Western blot :*

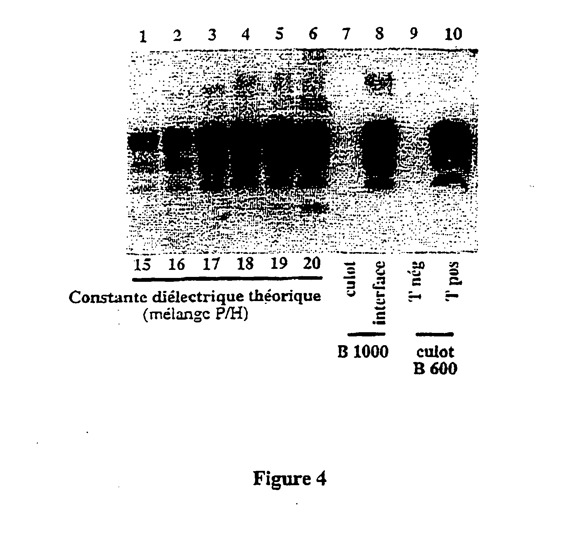

Les échantillons obtenus sont utilisés pour réaliser une électrophorèse SDS-PAGE et transférés sur membrane de nitrocellulose, dans les conditions décrites par Towbin et al. (Proc. Natl. Acad. Sci. USA, 1979, 76, 4350-4354) ou par C.I. Lasmézas et al. (J. Gen. Virol., 1996, précité).
Avant d'être déposés sur le gel d'électrophorèse, les échantillons ont été dilués au 1/20 dans un témoin négatif, produit dans les mêmes conditions que celles exposées à l'exemple 1. à partir d'un homogénat de vache saine, en raison de l'importance des signaux, (gel de polyacrylamide à 12 % chargé avec l'équivalent de 10 mg (10 μl) de cerveau, correspondant à 9.5 mg de cerveau de vache saine et 0.5 mg de cerveau de vache infectée).
L'immunodétection de la PrPres a été réalisée avec l'antisérum JB007 (R. Demaimay et al., J. Virol, 1997. 71, 12, 9685-9689) au 1/5 000ème, et des Ig de chèvre anti-lapin conjuguées à de la peroxydase (1/2500). L'immunoréactivité est révélée par chimiluminescence (ECL, Amersham), quantifiée et visualisée sur films autoradiographiques, comme illustré sur la figure 4.
La figure 4 illustre les résultats obtenus et correspond à la courbe propanol/hexanol de la figure 3A.
A cette figure, les pistes 1 à 6 correspondent à des échantillons traités en mettant en oeuvre comme tampon B. différents mélanges propanol/hexanol, conduisant à différents constantes diélectrique théoriques movennes ; les pistes 8 et 9 correspondent à des échantillons biologiques soumis à une procédure mettant en oeuvre comme tampon B, 1000 μl de butanol (point 53 % de la figure 1) : on observe dans ce cas que l'ensemble de la PrPres se retrouve à l'interface : les pistes 9 et 10 correspondant à des échantillons biologiques soumis à une procédure mettant en oeuvre comme tampon B. 600 μl de butanol (point 43 % de la figure 1) : on observe dans ce cas que l'ensemble de la PrPres se retrouve dans le culot (piste 10), alors que dans un témoin négatif, traité dans les mêmes conditions (piste 9), on n'observe aucun signal.

**EXEMPLE 7 : Dosage ELISA de la PrPres, à partir d'un échantillon obtenu conformément à l'invention.**

On prépare un échantillon, dans les conditions suivantes :

- On prélève 400 mg de cerveau bovin : on le broie et on l'homogénéise à 20 % dans une solution de glucose à 5 % (1.6 ml), dans les mêmes conditions que celles exposées à l'exemple 1.
- On incube, à 37°C pendant 10 min (2 fois 5 min avec agitation intermédiaire), 500 μl d'homogénat obtenu

avec 500 μl de tampon A comprenant du sarkosyl à 10 % et du Triton X 100 à 10 % ainsi que de la protéinase K (80 μg/ml).

- On ajoute 500 μl de tampon B (butanol).
- On centrifuge à 15 000 rpm avec un rotor permettant d'obtenir 17 608 g, pendant 5 min : les mêmes résultats sont obtenus avec un temps de centrifugation de 4 min à 20 627 g.
- On élimine le surnageant et on dissout le culot obtenu qui contient de la PrPres avec 100 μl de tampon C comprenant de l'urée 6 M et du sarkosyl 0,5 % pendant 5 min à 100°C.
- On centrifuge à 15 000 rpm avec un rotor permettant d'obtenir 17 608 g, pendant 5 min : les mêmes résultats sont obtenus avec un temps de centrifugation de 4 min à 20 627 g. Le surnageant est dilué au 1/3 dans du tampon EIA (tampon phosphate avec 0.5 % de désoxycholate).

Pour effectuer l'ELISA, l'échantillon obtenu est déposé à raison de 100 μl par puits, en duplicat sur une plaque recouverte avec un premier anticorps antiPrP (8G8, réf. Kraseman et al.. Molecular Medecine 1996), puis on incube, lave et révèle avec un deuxième anticorps anti-PrP (12F10, réf. Kraseman et al., 1996) couplé à l'acétylcholinestérase (réf. Grassi et al., J. Immunol Methods. 1989). On incube avec le substrat de l'acéwlcholine estérase puis la lecture se fait à 415 nm, 1 min ou 30 min. après ajout du substrat (réactif d'Ellmann).

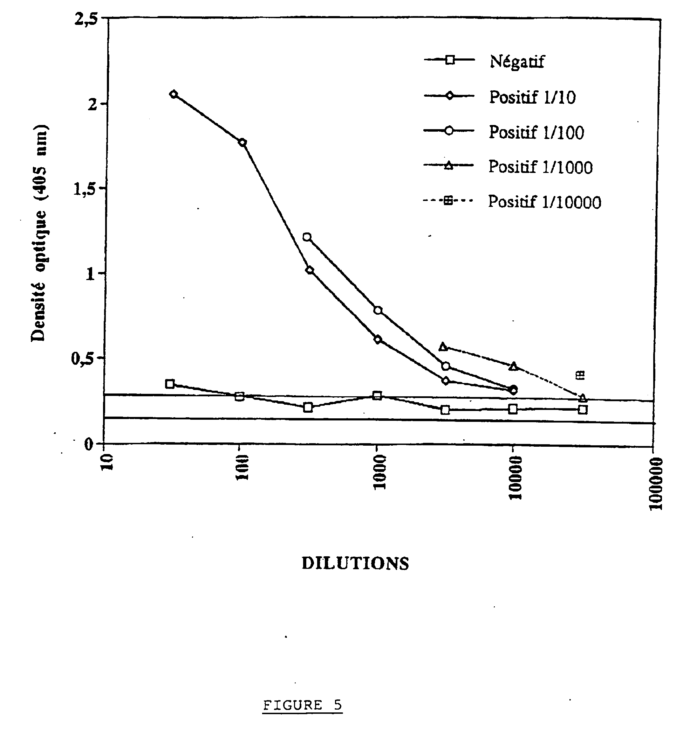

Les résultats présentés dans la figure 5 sont relatifs à un homogénat à 20 % (poids/volume) de cerveau de vache cliniquement atteinte de ESB. dilué au 1/10, 1/100. 1/1 000 ou 1/10 000 dans un homogénat à 20 % (poids:volume) d'un cerveau de vache saine.

Les échantillons sont alors traités dans les conditions décrites ci-dessus, et testés sur test EIA avec l'anticorps 8G8 en capture et l'anticorps 12F10 en anticorps de détection.

Les échantillons sont testés dilués dans le tampon de dépôt EIA (dilutions au 1/3, puis de 3 en 3).

La figure 5 illustre les résultats obtenus (densité optique du signal en fonction de la dilution finale de l'homogénat à savoir dilution dans l'homogénat négatif x dilution dans le tampon EIA) : la courbe semi-logarithmique présente un aspect sigmoïde, classiquement observé dans tous les dosages de type EIA. De plus, il est à noter une bonne superposition des courbes obtenues à partir des homogénats au 1/10, 1/100 et 1/1 000. Enfin le niveau de sensibilité du test permet de détecter ce cerveau positif dilué au 1/10 000.

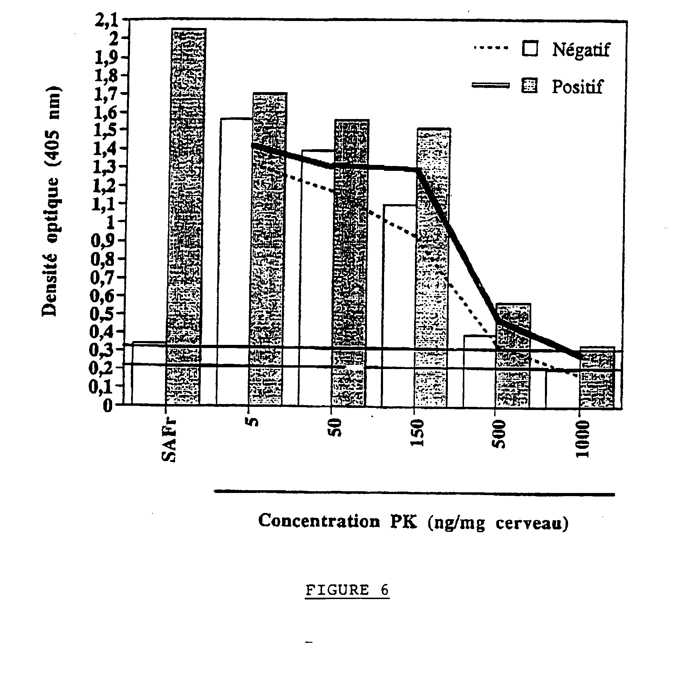

Dans la figure 6, un autre homogénat au 1/100 a été testé par technique EIA soit après traitement comme décrit ci-dessus, avec une concentration de PK à 400 ng/mg), soit après traitement des homogénats directement par différentes concentrations de PK. Il en ressort que le traitement direct des homogénats par la PK sans purification donne soit des signaux élevés des témoins négatifs pour de faibles concentrations de PK (destruction incomplète de PrPc), soit des signaux faibles pour les vaches positives avec des concentrations élevées de PK. Cet exemple souligne donc le caractère indispensable du traitement des homogénats tel qu'il a été décrit précédemment pour obtenir une détection correcte de la PrPres.

**EXEMPLE 8 : Comparaison de différents tampons C.**

Le protocole de traitement de l'échantillon utilisé est celui de l'exemple 7.

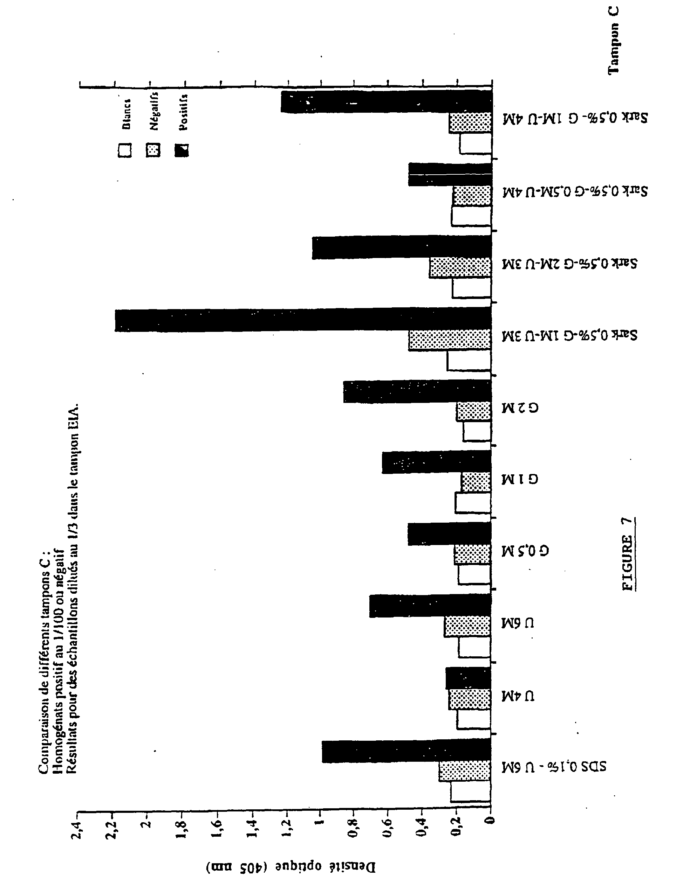

La figure 7 illustre les résultats obtenus.

La guanidine semble apporter un plus et la combinaison urée-guanidine parait la plus intéressante (sachant que l'urée est mieux supportée par le test ELISA).

**Revendications**

1. Procédé de purification de la protéine du prion sous une forme anormale (PrPres) à partir d'un échantillon biologique, **caractérisé en ce qu'**il comprend :

   (1) l'incubation, pendant 30 secondes à 2 heures, à une température inférieure à 80°C, dudit échantillon biologique avec un tampon A comprenant au moins un agent tensioactif en quantité comprise entre le quart et quatre fois le poids de l'échantillon biologique pour former une suspension S1 :
   (2) l'addition, à ladite suspension S1 obtenue en (1) d'un tampon B en quantité apte à éclaircir ladite suspension par formation d'une microémulsion ou d'une microsuspension, lequel tampon B est constitué d'un solvant ou d'un mélange de solvants, qui ne solubilise pas la PrPres et présente une constante diélectrique comprise entre 10 et 25 ;
   (3) la centrifugation de la suspension S2 obtenue à l'étape (2) ; et
   (4) la solubilisation dudit culot dans un tampon C comprenant au moins un agent tensioactif, du même type que celui de l'étape (1), à une concentration comprise entre 0,1 % et 5 %, de préférence comprise entre 0,25 % et 1%, par rapport au volume de tampon C (p/v) et/ou au moins un agent chaotrope à une concentration comprise entre 0,1 M et 8 M et à une température comprise entre la température ambiante et 100°C, de préférence égale ou supérieure à 80°C.

2. Procédé de purification de la protéine du prion sous une forme anormale (PrPres) à partir d'un échantillon biologique, **caractérisé en ce qu'**il comprend :

   (1) l'incubation, pendant 30 secondes à 2 heures, à une température inférieure à 80°C, dudit échantillon biologique avec un tampon A comprenant au moins un agent tensioactif en quantité comprise entre le quart et quatre fois le poids de l'échantillon biologique pour former une suspension S1 ;

(2) l'addition, à ladite suspension S1 obtenue en (1) d'un tampon B en quantité apte à former une séparation de phase, lequel tampon B est constitué d'un solvant ou d'un mélange de solvants, qui ne solubilise pas la PrPres et présente une constante diélectrique comprise entre 10 et 25 ;

(3) la centrifugation de la suspension obtenue à l'étape (2) ;

(4) la récupération du film présent à l'interface ;

(5) la resolubilisation dudit film avec un tampon A sans protéase,

(6) la centrifugation de la suspension obtenue à l'étape (5) ; et

(7) la solubilisation dudit culot dans un tampon C comprenant au moins un agent tensioactif, du même type que celui de l'étape (1), une concentration comprise entre 0,1 % et 5 %, de préférence comprise entre 0,25 % et 1 %, par rapport au volume de tampon C (p/v) et/ou au moins un agent chaotrope à une concentration comprise entre 0,1 M et 8 M et à une température comprise entre la température ambiante et 100°C, de préférence égale ou supérieure à 80°C,

3. Procédé selon la revendication 1 ou la revendication 2, **caractérisé en ce que** lorsque l'échantillon biologique est un tissu ou un organe, ce dernier est, préalablement à l'étape (1), homogénéisé dans un tampon d'homogénéisation, sélectionné dans le groupe constitué par les tampons neutres et les tampons isotoniques.

4. Procédé selon l'une quelconque des revendications 1 à 3, **caractérisé en ce que** la durée d'incubation est de préférence comprise entre 30 secondes à 10 minutes.

5. Procédé selon l'une quelconque des revendications 1 à 4, **caractérisé en ce que** le tampon A et/ou le tampon C comprend un agent tensioactif sélectionné dans le groupe constitué par :

- des tensioactifs anioniques, tels que le SDS (dodécylsulfate de sodium), le sarkosyl (lauroyl-sarcosine), le cholate de sodium, le désoxycholate de sodium, le taurocholate de sodium,
- des tensioactifs zwitterioniques, tels que le SB 3-10 (décylsulfobétaïne), le SB 3-12 (dodécyl-sulfobétaïne), le SB 3-14, le SB 3-16 (hexadécyl-sulfobétaïne), le CHAPS et le désoxy-CHAPS,
- des tensioactifs non-ioniques, tels que le C12E8 (dodécyloctaéthylèneglycol), le Triton X100, le Triton X114, le Tween 20, le Tween 80, le MEGA 9 (nonanoyl-méthylglucamine), l'octylglucoside, le LDAO (dodécyldiméthylamine oxyde) ou le NP40 ou

- des mélanges de tensioactifs tels qu'un mélange d'un agent tensioactif ionique et d'un agent tensioactif non-ionique, un mélange de deux agents tensioactifs ioniques ou un mélange d'un agent tensioactif ionique et d'un agent tensioactif zwitterionique.

6. Procédé selon l'une quelconque des revendications 1 à 5, **caractérisé en ce que** la température mise en oeuvre à l'étape (1) est comprise entre la température ambiante et 50°C, de préférence à 37°C.

7. Procédé selon l'une quelconque des revendications 1 à 5, **caractérisé en ce qu'**à l'étape (1), la quantité d'agent tensioactif présent dans le tampon A est de préférence comprise entre le quart et une fois et demi le poids de l'échantillon biologique.

8. Procédé selon l'une quelconque des revendications 1 à 7, **caractérisé en ce qu'**à l'étape (1) il comporte l'incubation de l'échantillon biologique avec une protéase, de façon préalable, postérieure ou simultanée à l'incubation dudit échantillon biologique avec le tampon A.

9. Procédé selon l'une quelconque des revendications 1 à 8, **caractérisé en ce que** le tampon B est sélectionné dans le groupe constitué par les alcools en C3-C6 et les mélanges d'alcools dont la constante diélectrique théorique moyenne est comprise entre 10 et 25.

10. Procédé selon la revendication 9, **caractérisé en ce que** les alcools ou mélanges d'alcools suivants sont particulièrement préférés : butanol-1, butanol-2, méthyl-2 propanol-1, isopropanol, isopropanol + pentanol, éthanol + hexanol, butanol + pentanol.

11. Procédé selon l'une quelconque des revendications 1 à 10, **caractérisé en ce que** la centrifugation de l'étape (3) et de l'étape (5) est réalisée pendant 2 à 10 minutes à une vitesse inférieure à 20 000 g, de préférence à une vitesse comprise entre 3 500 g et 17 500 g.

12. Procédé selon l'une quelconque des revendications 1 à 11, **caractérisé en ce que** le tampon C comprend comme agent chaotrope, un agent qui est sélectionné dans le groupe constitué par l'urée et la guanidine ou un mélange de ceux-ci,

13. Procédé selon l'une quelconque des revendications 1 à 12, **caractérisé en ce que** l'étape de solubilisation (étape (4) du procédé selon la revendication 1 ou étape (7) du procédé selon la revendication 2) dans ledit tampon C comprend le chauffage à une température égale ou supérieure à 80°C, pendant 5 à 10 minutes, suivie d'une centrifugation.

14. Procédé de détection de la PrPres dans un échantillon biologique, **caractérisé en ce qu'**il comprend :

    - le traitement dudit échantillon selon l'une quelconque des revendications 1 à 13, et
    - la détection de la PrPres par toute méthode analytique appropriée.

15. Procédé selon la revendication 14 **caractérisé en ce qu'**il comprend une étape de dilution de l'échantillon biologique.

16. Kit de traitement d'un échantillon biologique, en vue de la purification de la PrPres à partir dudit échantillon, **caractérisé en ce qu'**il comprend, outre un tampon neutre en quantité permettant l'homogénéisation dudit échantillon biologique :

    - un tampon A comprenant au moins un agent tensioactif en quantité comprise entre le quart et quatre fois le poids de l'échantillon biologique,
    - un tampon B constitué d'un solvant ou d'un mélange de solvants, qui ne solubilise pas la PrPres et présente une constante diélectrique comprise entre 10 et 25, en quantité apte à éclaircir une suspension issue de l'incubation de l'échantillon biologique dans le tampon A ;
    - un tampon C comprenant au moins un agent tensioactif, du même type que celui du tampon A, à une concentration comprise entre 0,1 % et 5 %, de préférence comprise entre 0,25 % et 1%, par rapport au volume de tampon C (p/v) et/ou au moins un agent chaotrope à une concentration comprise entre 0,1 M et 8 M, en quantité suffisante pour permettre la solubilisation de la PrPres.

17. Kit de détection de la PrPres, **caractérisé en ce qu'**il comprend :

    - un tampon neutre en quantité permettant l'homogénéisation de l'échantillon biologique dans lequel la PrPres doit être détectée,
    - un tampon A comprenant au moins un agent tensioactif en quantité comprise entre le quart et quatre fois le poids de l'échantillon biologique,
    - un tampon B constitué d'un solvant ou d'un mélange de solvants, qui ne solubilise pas la PrPres et présente une constante diélectrique comprise entre 10 et 25, en quantité apte à éclaircir une suspension issue de l'incubation de l'échantillon biologique dans le tampon A ;
    - un tampon C comprenant au moins un agent tensioactif, du même type que celui du tampon A, à une concentration comprise entre 0,1 % et 5 % , de préférence comprise entre 0,25 % et 1 %, par rapport au volume de tampon C (p/v) et/ou au moins un agent chaotrope à une concentration comprise entre 0,1 M et 8 M, en quantité suffisante pour permettre la solubilisation de la PrPres.
    - au moins un anticorps anti-PrPres permettant la détection de la PrPres.

## Claims

1. Method of purifying abnormal form of prion protein (PrPres) from a biological sample, **characterized in that** it comprises:

    (1) the incubation, for 30 seconds to 2 hours, at a temperature below 80°C, of said biological sample with a buffer A comprising at least one surfactant in an amount of between a quarter and four times the weight of the biological sample, to form a suspension S1;
    (2) the addition, to said suspension S1 obtained in (1), of a buffer B in an amount suitable for clarifying said suspension by forming a microemulsion or a microsuspension, said buffer B consisting of a solvent or solvent mixture which does not solubilize the PrPres and has a dielectric constant of between 10 and 25;
    (3) the centrifugation of the suspension S2 obtained in step (2); and
    (4) the solubilization of said residue in a buffer C comprising at least one surfactant, as defined in step (1), at a concentration of between 0.1% and 5%, preferably of between 0.25% and 1%, based on the volume of buffer C (w/v), and/or at least one chaotropic agent at a concentration of between 0,1 M and 8 M, and at a temperature between room temperature and 100°C, preferably equal to or greater than 80°C.

2. Method of purifying abnormal form of prion protein (PrPres) from a biological sample, **characterized in that** it comprises:

    (1) the incubation, for 30 seconds to 2 hours, at a temperature below 80°C, of said biological sample with a buffer A comprising at least one surfactant in an amount of between a quarter and four times the weight of the biological sample, to form a suspension S1;
    (2) the addition, to said suspension S1 obtained in (1), of a buffer B in an amount suitable for creating a phase separation, said buffer B consisting of a solvent or solvent mixture which does not solubilize the PrPres and has a dielectric constant of between 10 and 25;

(3) the centrifugation of the suspension obtained in step (2);

(4) the recovery of the film present at the interface;

(5) the resolubilization of said film with a buffer A without the addition of protease;

(6) the centrifugation of the suspension obtained in step (5); and

(7) the solubilization of said residue in a buffer C comprising at least one surfactant, as defined in step (1), at a concentration of between 0.1% and 5%, preferably of between 0.25% and 1%, based on the volume of buffer C (w/v), and/or at least one chaotropic agent at a concentration of between 0,1 M and 8 M, and at a temperature between room temperature and 100°C, preferably equal to or greater than 80°C.

3. Method according to claim 1 or claim 2, **characterized in that**, when the biological sample is a tissue or an organ, it is homogenized prior to step (1) in a homogenization buffer selected from the group consisting of neutral buffers and isotonic buffers.

4. Method according to any one of claims 1 to 3, **characterized in that** the incubation time is preferably between 30 seconds and 10 minutes.

5. Method according to any one of claims 1 to 4, **characterized in that** buffer A and/or buffer C comprise a surfactant selected from the group consisting of:

    - anionic surfactants such as SDS (sodium dodecylsulfate), sarkosyl (lauroylsarcosine), sodium cholate, sodium deoxycholate, sodium taurocholate;
    - zwitterionic surfactants such as SB 3-10 (decyl sulfobetaine), SB 3-12 (dodecyl sulfobetaine), SB 3-14, SB 3-16 (hexadecyl sulfobetaine), CHAPS or deoxyCHAPS;
    - non-ionic surfactants such as C12E8 (dodecyl octaethylene glycol), Triton X100, Triton X114, Tween 20, Tween 80, MEGA 9 (nonanoylmethylglucamine), octylglucoside, LDAO (dodecyldimethylamine oxide) or NP40; or
    - surfactant mixtures such as a mixture of an ionic surfactant and a non-ionic surfactant, a mixture of two ionic surfactants or a mixture of an ionic surfactant and a zwitterionic surfactant.

6. Method according to any one of claims 1 to 5, **characterized in that** the temperature used in step (1) is between room temperature and 50°C, preferably 37°C.

7. Method according to any one of claims 1 to 5, **characterized in that**, in step (1), the amount of surfactant present in buffer A is preferably between a quarter and one and a half times the weight of the biological sample.

8. Method according to any one of claims 1 to 7, **characterized in that**, in step (1) it comprises the incubation of the biological sample with a protease, prior to, after or simultaneously with, the incubation of said biological sample with buffer A.

9. Method according to any one of claims 1 to 8, **characterized in that** buffer B is selected from the group consisting of $C_3$-$C_6$ alcohols and alcohols mixtures with a mean theoretical dielectric constant of between 10 and 25.

10. Method according to claim 9, **characterized in that** the following alcohols or alcohol mixtures are particularly preferred: butan-1-ol, butan-2-ol, 2-methylpropan-1-ol, isopropanol, isopropanol + pentanol, ethanol + hexanol and butanol + pentanol.

11. Method according to any one of claims 1 to 10, **characterized in that** the centrifugation of step (3) and step (5) is carried out for 2 to 10 minutes at a speed below 20,000 g, preferably at a speed of between 3,500 g and 17,500 g.

12. Method according to any one of claims 1 to 11, **characterized in that** buffer C comprises a chaotropic agent which is selected from the group consisting of urea and guanidine or a mixture thereof.

13. Method according to any one of claims 1 to 12, **characterized in that** the solubilization step (step (4) of the method according to claim 1 or step (7) of the method according to claim 2) in said buffer C comprises heating at a temperature equal to or greater than 80°C for 5 to 10 minutes, followed by centrifugation.

14. Method of detecting PrPres in a biological sample, **characterized in that** it comprises:

    - treating said sample according to any one of claims 1 to 13, and
    - detecting the PrPres by any appropriate analytical method.

15. Method according to claim 14, **characterized in that** it comprises a dilution step of the biological sample.

16. Kit for treating a biological sample for the purpose of purifying PrPres from said biological sample, **characterized in that** it comprises, in addition to a neutral buffer in an amount suitable for homogenizing said biological sample:

- a buffer A comprising at least one surfactant in an amount of between a quarter and four times the weight of the biological sample,
- a buffer B consisting of a solvent or solvent mixture which does not solubilize the PrPres and has a dielectric constant of between 10 and 25, in an amount suitable for clarifying a suspension derived from the incubation of the biological sample with buffer A;
- a buffer C comprising at least one surfactant, as defined in buffer A, at a concentration of between 0.1% and 5%, preferably of between 0.25% and 1%, based on the volume of buffer C (w/v), and/or at least one chaotropic agent at a concentration of between 0,1 M and 8 M, in an amount suitable for solubilizing PrPres.

17. PrPres detection kit, **characterized in that** it comprises:

- a neutral buffer in an amount suitable for homogenizing the biological sample in which the PrPres is to be detected,
- a buffer A comprising at least one surfactant in an amount of between a quarter and four times the weight of the biological sample,
- a buffer B consisting of a solvent or solvent mixture which does not solubilize the PrPres and has a dielectric constant of between 10 and 25, in an amount suitable for clarifying a suspension derived from the incubation of the biological sample with buffer A;
- a buffer C comprising at least one surfactant, as defined in buffer A, at a concentration of between 0.1 % and 5%, preferably of between 0.25% and 1%, based on the volume of buffer C (w/v), and/or at least one chaotropic agent at a concentration of between 0,1 M and 8 M, in an amount suitable for solubilizing PrPres,
- at least one anti-PrPres antibody for detecting PrPres.

**Patentansprüche**

1. Verfahren zur Reinigung einer abnormen Form des Prion-Proteins (PrPres) aus einer biologischen Probe, **dadurch gekennzeichnet, dass** es umfasst:

(1) Inkubation der biologischen Probe für 30 Sekunden bis 2 Stunden bei einer Temperatur unter 80 °C mit einem Puffer A, der wenigstens ein Tensid in einer Menge zwischen einem Viertel und dem Vierfachen des Gewichts der biologischen Probe umfasst, unter Bildung einer Suspension S1;
(2) Zugabe eines Puffers B zu der in (1) erhaltenen Suspension S1 in einer Menge, die geeignet ist, diese Suspension durch Bildung einer Mikroemulsion oder einer Mikrosuspension klar zu machen, wobei der Puffer B aus einem Lösungsmittel oder einer Lösungsmittelmischung besteht, die das PrPres nicht solubilisieren und eine Dielektrizitätskonstante zwischen 10 und 25 haben;
(3) Zentrifugation der in Schritt (2) erhaltenen Suspension S2; und
(4) Solubilisierung des Pellets in einem Puffer C, der wenigstens ein Tensid des gleichen Typs wie in Schritt (1) in einer Konzentration zwischen 0,1 % und 5 %, vorzugsweise zwischen 0,25 % und 1 %, bezogen auf das Volumen von Puffer C (w/v), und/oder wenigstens ein chaotropes Mittel in einer Konzentration zwischen 0,1 M und 8 M umfasst, und bei einer Temperatur zwischen Raumtemperatur und 100 °C, vorzugsweise gleich oder über 80 °C.

2. Verfahren zur Reinigung der abnormen Form des Prion-Proteins (PrPres) aus einer biologischen Probe, **dadurch gekennzeichnet, dass** es umfasst:

(1) Inkubation der biologischen Probe für 30 Sekunden bis 2 Stunden bei einer Temperatur unter 80 °C mit einem Puffer A, der wenigstens ein Tensid in einer Menge zwischen einem Viertel und dem Vierfachen des Gewichts der biologischen Probe umfasst, unter Bildung einer Suspension S1;
(2) Zugabe eines Puffers B zu der in (1) erhaltenen Suspension S1 in einer Menge, die geeignet ist, eine Phasentrennung herbeizuführen, wobei der Puffer B aus einem Lösungsmittel oder einer Lösungsmittelmischung besteht, die das PrPres nicht solubilisieren und eine Dielektrizitätskonstante zwischen 10 und 25 haben;
(3) Zentrifugation der in Schritt (2) erhaltenen Suspension;
(4) Gewinnung des an der Phasengrenzfläche vorliegenden Films;
(5) Resolubilisierung des Films mit einem Puffer A ohne Protease;
(6) Zentrifugation der in Schritt (5) erhaltenen Suspension; und
(7) Solubilisierung des Pellets in einem Puffer C, der wenigstens ein Tensid des gleichen Typs wie in Schritt (1) in einer Konzentration zwischen 0,1 % und 5 %, vorzugsweise zwischen 0,25 % und 1 %, bezogen auf das Volumen von Puffer C (w/v), und/oder wenigstens ein chaotropes Mittel in einer Konzentration zwischen 0,1 M und 8 M umfasst, und bei einer Temperatur zwischen Raumtemperatur und 100 °C, vorzugsweise gleich oder über 80 °C.

3. Verfahren nach Anspruch 1 oder Anspruch 2, **da-**

**durch gekennzeichnet, dass** wenn die biologische Probe ein Gewebe oder ein Organ ist, letzteres zuvor in Schritt (1) in einem Homogenisierungspuffer homogenisiert wird, der ausgewählt ist aus der Gruppe bestehend aus neutralen Puffern und isotonischen Puffern.

4. Verfahren nach irgendeinem der Ansprüche 1 bis 3, **dadurch gekennzeichnet, dass** die Inkubationsdauer vorzugsweise zwischen 30 Sekunden und 10 Minuten liegt.

5. Verfahren nach irgendeinem der Ansprüche 1 bis 4, **dadurch gekennzeichnet, dass** der Puffer A und/ oder der Puffer C ein Tensid umfassen, das ausgewählt ist aus der Gruppe bestehend aus:

  - anionischen Tensiden wie SDS (Natriumdodecylsulfat), Sarkosyl (Lauroylsarcosin), Natriumcholat, Natriumdesoxycholat, Natriumtaurocholat,
  - zwitterionischen Tensiden wie SB 3-10 (Decyl-Sulfobetain), SB 3-12 (Dodecyl-Sulfobetain), SB 3-14, SB 2-16 (Hexadecyl-Sulfobetain), CHAPS und DeoxyCHAPS,
  - nichtionischen Tensiden wie C12E8 (Dodecyloctaethylenglycol), Triton X100, Triton X114, Tween 20, Tween 80, MEGA 9 (Nonanoylmethylglucamin) Octylglucosid, LDAO (Dodecyldimethylaminoxid) oder NP40, oder
  - Mischungen von Tensiden, wie eine Mischung aus einem ionischen Tensid und einem nichtionischen Tensid, eine Mischung aus zwei ionischen Tensiden oder eine Mischung aus einem ionischen Tensid und einem zwitterionischen Tensid.

6. Verfahren nach irgendeinem der Ansprüche 1 bis 5, **dadurch gekennzeichnet, dass** die in Schritt (1) angewandte Temperatur zwischen Raumtemperatur und 50 °C, vorzugsweise bei 37 °C liegt.

7. Verfahren nach irgendeinem der Ansprüche 1 bis 5, **dadurch gekennzeichnet, dass** in Schritt (1) die Menge an Tensid in Puffer A vorzugsweise zwischen einem Viertel und dem Eineinhalbfachen des Gewichts der biologischen Probe liegt.

8. Verfahren nach irgendeinem der Ansprüche 1 bis 7, **dadurch gekennzeichnet, dass** es in Schritt (1) vor, nach oder gleichzeitig mit der Inkubation der biologischen Probe mit Puffer A die Inkubation der biologischen Probe mit einer Protease beinhaltet.

9. Verfahren nach irgendeinem der Ansprüche 1 bis 8, **dadurch gekennzeichnet, dass** Puffer B ausgewählt ist aus der Gruppe bestehend aus $C_3$-$C_6$-Alkoholen und Alkoholmischungen mit einer mittleren theoretischen Dielektrizitätskonstante zwischen 10 und 25.

10. Verfahren nach Anspruch 9, **dadurch gekennzeichnet, dass** die folgenden Alkohole oder Alkoholmischungen besonders bevorzugt sind: 1-Butanol, 2-Butanol, 2-Methyl-1-propanol, Isopropanol, Isopropanol + Pentanol, Ethanol + Hexanol, Butanol + Pentanol.

11. Verfahren nach irgendeinem der Ansprüche 1 bis 10, **dadurch gekennzeichnet, dass** die Zentrifugation in Schritt (3) und in Schritt (5) 2 bis 10 Minuten lang bei einer Geschwindigkeit unter 20.000 g, vorzugsweise bei einer Geschwindigkeit zwischen 3.500 g und 17.500 g durchgeführt wird.

12. Verfahren nach irgendeinem der Ansprüche 1 bis 11, **dadurch gekennzeichnet, dass** der Puffer C als chaotropes Mittel ein Mittel umfasst, das ausgewählt ist aus der Gruppe bestehend aus Harnstoff und Guanidin oder einer Mischung davon.

13. Verfahren nach irgendeinem der Ansprüche 1 bis 12, **dadurch gekennzeichnet, dass** der Solubilisierungsschritt (Schritt (4) des Verfahrens nach Anspruch 1 oder Schritt (7) des Verfahrens nach Anspruch 2) in Puffer C das Erwärmen für 5 bis 10 Minuten auf eine Temperatur gleich oder höher 80 °C umfasst, gefolgt von einer Zentrifugation.

14. Verfahren zum Nachweis von PrPres in einer biologischen Probe, **dadurch gekennzeichnet, dass** es umfasst:

  - Behandlung der Probe gemäß irgendeinem der Ansprüche 1 bis 13 und
  - Nachweis von PrPres mit einem geeigneten Analyseverfahren.

15. Verfahren nach Anspruch 14, **dadurch gekennzeichnet, dass** es einen Verdünnungsschritt für die biologische Probe umfasst.

16. Kit zur Behandlung einer biologischen Probe zur Reinigung von PrPres aus dieser Probe, **dadurch gekennzeichnet, dass** er neben einem neutralen Puffer in einer die Homogenisierung der biologischen Probe ermöglichenden Menge umfasst:

  - einen Puffer A, der wenigstens ein Tensid in einer Menge zwischen einem Viertel und dem Vierfachen des Gewichts der biologischen Probe umfasst,
  - einen Puffer B, der aus einem Lösungsmittel oder einer Lösungsmittelmischung besteht, die das PrPres nicht solubilisieren und eine Dielektrizitätskonstante zwischen 10 und 25 haben, in einer Menge, die geeignet ist, eine bei der Inkubation der biologischen Probe in Puffer A entstandene Suspension klar zu machen,

- einen Puffer C, der wenigstens ein Tensid des gleichen Typs wie in Puffer A in einer Konzentration zwischen 0,1 % und 5 %, vorzugsweise zwischen 0,25 % und 1 %, bezogen auf das Volumen von Puffer C (w/v), und/oder wenigstens ein chaotropes Mittel in einer Konzentration zwischen 0,1 M und 8 M umfasst, in einer für die Solubilisierung des PrPres ausreichenden Menge.

**17.** Kit zum Nachweis von PrPres, **dadurch gekennzeichnet, dass** er umfasst:

- einen neutralen Puffer in einer Menge, die die Homogenisierung der biologischen Probe erlaubt, in der das PrPres nachgewiesen werden soll,

- einen Puffer A, der wenigstens ein Tensid in einer Menge zwischen einem Viertel und dem Vierfachen des Gewichts der biologischen Probe umfasst,

- einen Puffer B, der aus einem Lösungsmittel oder einer Lösungsmittelmischung besteht, die das PrPres nicht solubilisieren und eine Dielektrizitätskonstante zwischen 10 und 25 haben, in einer Menge, die geeignet ist, eine bei der Inkubation der biologischen Probe in Puffer A entstandene Suspension klar zu machen,

- einen Puffer C, der wenigstens ein Tensid des gleichen Typs wie in Puffer A in einer Konzentration zwischen 0,1 % und 5 %, vorzugsweise zwischen 0,25 % und 1 %, bezogen auf das Volumen von Puffer C (w/v), und/oder wenigstens ein chaotropes Mittel in einer Konzentration zwischen 0,1 M und 8 M umfasst, in einer für die Solubilisierung des PrPres ausreichenden Menge,

- wenigstens einen anti-PrPres-Antikörper, der den Nachweis des PrPres erlaubt.

FIGURE 1

FIGURE 2

FIGURE 3A

FIGURE 3B

Figure 4

**DILUTIONS**

FIGURE 5

FIGURE 6

Comparaison de différents tampons C :
Homogénats positif au 1/100 ou négatif
Résultats pour des échantillons dilués au 1/3 dans le tampon EIA.

FIGURE 7

Tampon C